Europäisches Patentamt

European Patent Office

Office européen des brevets

(19)

(11) EP 0 614 464 B1

(12) EUROPEAN PATENT SPECIFICATION

(45) Date of publication and mention
of the grant of the patent:
**11.03.1998 Bulletin 1998/11**

(51) Int Cl.[6]: **C07K 5/08**, C07K 5/10,
C07K 7/06, C12P 21/08,
A61K 38/04

(21) Application number: **92923909.3**

(22) Date of filing: **25.11.1992**

(86) International application number:
**PCT/GB92/02174**

(87) International publication number:
**WO 93/11153 (10.06.1993 Gazette 1993/14)**

(54) **PEPTIDES AND ANTIBODIES FOR TREATMENT OF RHEUMATOID ARTHRITIS**

Peptide und Antikörper zur Behandlung von rheumatoider Arthritis.

PEPTIDES ET ANTICORPS UTILISES DANS LE TRAITEMENT DE LA POLYARTHRITE RHUMATOIDE

(84) Designated Contracting States:
**AT BE CH DE DK ES FR GR IE IT LI LU MC NL PT SE**

(30) Priority: **25.11.1991 GB 9125024**

(43) Date of publication of application:
**14.09.1994 Bulletin 1994/37**

(73) Proprietor: **PEPTIDE THERAPEUTICS LIMITED
Cambridge CB4 4NG (GB)**

(72) Inventors:
• **KIRBY, Julian
Burton-upon-Trent Staffordshire DE13 0RQ (GB)**
• **LEWIN, Ian, Victor
Tamworth Staffordshire B77 3PE (GB)**
• **MAMAN, Sarita
Wolverhamton West Midlands WV4 5SE (GB)**
• **STANWORTH, Denis, Raymond
Birmingham B29 7JQ (GB)**

(74) Representative: **Davies, Jonathan Mark et al
Reddie & Grose
16 Theobalds Road
London WC1X 8PL (GB)**

(56) References cited:
WO-A-88/00240          WO-A-90/12806
WO-A-90/13573          US-A- 4 692 511

• PROCEEDINGS OF THE NATIONAL ACADEMY OF SCIENCES, USA vol. 88, 1991, WASHINGTON, USA pages 2648 - 2652 FUNK ET AL 'The Ca2+-binding glycoprotein SPARC modulates cell cycle progression in bovine aortic endothelial cells'
• PROCEEDINGS OF THE NATIONAL ACADEMY OF SCIENCES, USA vol. 85, July 1988, WASHINGTON, USA pages 5181 - 5185 ADORINI ET AL 'Interaction of an immunodominant epitope with Ia molecules in T-cell activation'
• CHEM. PHARM. BULL vol. 31, no. 9, 1983, TOKYO, JAPAN pages 3094 - 3103 OHTA ET AL 'Amino acids and Peptides. IX. Synthesis of cysteine-containing peptide fragments related to human hepatic metallothionein II (hMT II) and determination of their heavy metal-binding properties'
• CHEMICAL ABSTRACTS, vol. 81, 1974, Columbus, Ohio, US; abstract no. 103073, TOMASI ET AL 'Binding of alpha-1 antitrypsin to human IgA' page 340 ;column 1 ;
• CHEMICAL ABSTRACTS, vol. 96, 1982, Columbus, Ohio, US; abstract no. 215728, OHTANI ET AL 'Protein contents and binding modes of immunoglobulin-amylase complexes' page 556 ;column 2 ;
• CHEMICAL ABSTRACTS, vol. 116, 1992, Columbus, Ohio, US; abstract no. 124390, STANWORTH ET AL 'immunodiagnostic assay for rheumatoid arthritis' page 437 ;column 2 ;

**Description**

Field of the invention

This invention is in the field of rheumatoid arthritis (RA) treatment.

Description of the prior art

Rheumatoid arthritis, (RA), has been described as an unresolved systemic inflammation in which immune dysfunction and genetic susceptibility play roles. There is increasing evidence that the major immunopathological factor in RA is the covalently linked complex between serum IgA and $\alpha_1$-antitrypsin ($\alpha_1$AT), a major anti-protease. This disulphide-bridged complex is found to be present at abnormally high levels in the sera and joint fluids of RA patients. In vitro studies have revealed that the complex is capable of inducing the release of tissue-degradative enzymes from mouse macrophages, whilst injection of the complex itself into the knee joints of rabbits or mice causes the onset of RA like arthritis.

It has been shown that a fall in the circulating level of the complex is observed in those rheumatoid patients who respond favourably to treatment with so-called "second-line" anti-rheumatic drugs such as D-penicillamine (dimethyl cysteine) and myocrisin (sodium aurothiomalate). This can be explained by these thiol compounds forming mixed disulphides with the thiol-active IgA produced in large amounts by rheumatoid patients, thereby preventing its reaction with $\alpha_1$-antitrypsin to form the undesired IgA-$\alpha_1$AT complex (Stanworth, D. R. Immunology Today 1985, 6, 43) .

There is a problem with the currently available thiol-based anti-rheumatic drugs such as D-penicillamine in that they are relatively toxic, causing many rheumatoid patients to end their treatment prematurely.

It is therefore desirable to find alternative means of combatting rheumatoid arthritis.

WO 90/12806 (Neosystem S.A.) discloses certain immunogenic synthetic peptides which are formed as a conjugate of (i) a sequence which is a fraction of the sequence of ubiquintine (e.g. Gly-Gly-Arg-Leu) and (ii) a sequence which is a fraction of the sequence of histone H2A (e.g. Lys-Lys-Thr-Glu). These branched chain amino acid conjugates may induce antibody production and may be useful in diagnosis or treatment of autoimmune disease.

Galpin et al in Tetrahedron Vol. 37, No. 17, 3025-3029 discloses a fragment of lysozyme analogue (compound 121) having the sequence including Cys-Ala-Lys-Lys-Ile.

Summary of the Invention

RA patients are known to produce grossly elevated levels of thiol-reactive IgA which is thought to be covalently bonded to $\alpha_1$AT to form the IgA-$\alpha_1$AT complex. It has now been found that certain peptides, fulfilling certain specified requirements as to their charged amino acid groups and capable of interacting with thiol-reactive IgA at a thiol-reactive cysteine residue, are capable of dissociating IgA-$\alpha_1$AT complex or preventing IgA-$\alpha_1$AT complex formation. Dissociation of complex or prevention of complex formation might be expected to have a favourable effect against clinical RA and is therefore of therapeutic potential. Monoclonal antibodies raised against IgA-$\alpha_1$AT complex are also capable of alleviating the effects of IgA-$\alpha_1$AT complex in RA patients.

Accordingly, the invention relates to pharmaceutical compositions comprising a synthetic peptide of from 3 to 7 amino acid residues or an analogue thereof wherein the analogue is at least partly non-peptide in nature comprising a single thiol-active cysteine residue,
the peptide or analogue thereof being capable of preventing the formation of or dissociating already formed IgA-$\alpha_1$AT complex, for use in treatment of rheumatoid arthritis.

Terminal functions of the peptide can be blocked, e.g. by N-acylation or C-amidation, or the peptides can be derivatised in any conventional manner.

Brief Description of the Drawings

Figure 1 shows the results of an experiment to determine the causative agent of joint erosions in rabbits, in which (A) shows the results for IgA injected rabbits, (B) shows the results for synovial fluid injected rabbits and (C) shows the results for IgA-$\alpha_1$AT injected rabbits.

Figure 2 shows the results of an experiment in which either a peptide composition of the invention or a placebo was administered to arthritic rabbits.

Description of the preferred embodiments

The compositions of the invention are those in which there is a thiol-active cysteine residue and at least two positively charged amino acids. For convenience in this specification, both the one-letter code and the three-letter code for amino acids are used when discussing peptides. The one-letter code and three-letter code is shown below.

|  | Three-letter | One-letter |
|---|---|---|
| Amino acid | Symbol | Symbol |
| Alanine | Ala | A |
| Arginine | Arg | R |
| Asparagine | Asn | N |
| Aspartic acid | Asp | D |
| Asn and/or Asp | Asx | B |
| Cysteine | Cys | C |
| Glutamine | Gln | Q |
| Glutamic acid | Glu | E |
| Gln and/or Glu | Glx | Z |
| Glycine | Gly | G |
| Histidine | His | H |
| Isoleucine | Ile | I |
| Leucine | Leu | L |
| Lysine | Lys | K |
| Methionine | Met | M |
| Phenylalanine | Phe | F |
| Proline | Pro | P |
| Serine | Ser | S |
| Threonine | Thr | T |
| Tryptophan | Trp | W |
| Tyrosine | Tyr | Y |
| Valine | Val | V |

Among the 20 commonest amino acids, the positively charged amino acids are those which are basic and are arginine, lysine or histidine. Negatively charged amino acids are those which are acidic and are aspartic acid and glutamic acid. Neutral amino acids do not carry an overall positive or negative charge and are the remaining amino acids. Of course, uncommon amino acids or derivatives of common amino acids could alternatively be employed in place of the 20 commonest.

The invention provides:

a pharmaceutical composition comprising a prophylactically or therapeutically effective amount of a synthetic peptide consisting of from 3 to 7 amino acid residues or analogue thereof wherein the analogue is at least partly non-peptide in nature comprising a single thiol-active cysteine residue and having the following general formula:

$$Z^1_a\text{-}B^1_c\text{-}Cys\text{-}B^2_d\text{-}Lys\text{-}Lys\text{-}Z^2_b$$

Wherein a positively charged amino acid residue is located on the peptide's N-terminal or C-terminal; and wherein $Z^1$, $Z^2$, $B^1$ and $B^2$ represent sequences of positively charged, negatively charged or neutral amino acid residues or sequences of any mixture of positively charged, negatively charged or neutral amino acid residues; or $B^1$ and $B^2$ represent non-peptide spacer arms of a length equivalent to that determined by the length of d and c residues of amino acids c = 0 to 4, d = 0 to 4; and

a = 0 to 4 and b = 0 to 4 and a + b + c + d ≤ 4;

the peptide or analogue thereof being capable of preventing the formation of or dissociating already formed $IgA\text{-}\alpha_1AT$ complex, with the proviso that the peptide does not include the sequence Cys-Ala-Lys-Lys-Ile or Cys-Lys-Lys-Thr-Glu.

The compositions of the invention are particularly useful for the prevention or treatment of RA. They are effective since they are capable of interacting with cysteine residues within the thiol-active IgA and thus preventing $\alpha_1AT$ binding or dislodging $\alpha_1AT$ already bound.

The peptides must include sufficient amino acid residues on one or both sides of the thiol-reactive cysteine residues to ensure that the resultant peptide will form a mixed disulphide with thiol-reactive (rheumatoid) IgA which thus stabilises the immunoglobulin in an <u>essentially native configuration</u> and does not allow its covalent complexing to $\alpha_1$-antitrypsin.

The length of the peptide is from 3 to 7.

The shorter peptides are preferred since there is a minimal risk of the host mounting an immune response against the peptide of the invention.

The amino acids in the spacer arm are not cysteine. Preferably the amino acids are of neutral charge and more preferably are glycine. More preferably, the positively charged amino acid residues are directly adjacent to the cysteine residue on the N-terminal, the C-terminal or both the N- and C-terminals.

Preferred peptides are those which consist of or include (1) residues 231-233 of $\alpha_1$AT or analogues thereof, i.e. His-Cys-Lys, or (ii) residues 232-234 of $\alpha_1$AT or analogues thereof, i.e. Cys-Lys-Lys. More preferably, the peptides comprise the residues 231-234 of $\alpha_1$AT or analogues thereof, i.e. His-Cys-Lys-Lys.

The peptide used in the invention is preferably amidated at the C-terminal. The effect of this amidation is to prolong the half-life of the peptide, thus enabling shorter peptides to be used, and increasing the anti-rheumatic activity. Preferably where the N-terminal is a positively charged amino acid residue the N-terminal is not acylated.

Mixtures or D and L amino acids may be used, or the peptide may exculsively contain D-residues or exclusively contain L-residues.

The peptides may be used both prophylactically and therapeutically, GB 2246780 describes a method of diagnosing patients suffering from RA as opposed to other diseases which may manifest themselves with similar symptoms. This method may also be used to detect RA in patients who have not yet developed joint erosions (so called "early" RA patients). This method measures the amount of circulating IgA-$\alpha_1$AT complex. By the constant monitoring of early RA patients, therefore, the normal level of complex in that individual can be ascertained, and the amount of peptide administered calculated accurately, depending on the level of circulating complex above a normal value in a particular patients. The peptides in early RA patients are preferably administered prophylactically (i.e. to prevent formation of elevated levels of IgA-$\alpha_1$AT complex). In patients who have developed joint erosions already, it is too late for prophylactic administration of peptides and thus they are administered in a therapeutic sense, the dosages depending on the level of complex found circulating in those patients, and the value considered normal for that particular patient as determined by knowledge of what was his "normal" level of complex before RA developed, or at what concentration of complex are the symptoms of RA alleviated.

The peptides for use in therapy may be formulated with a physiologically acceptable diluent or carrier for use as pharmaceuticals for human use by a variety of methods. They may for instance be incorporated into a form suitable for injection or infusion and are therefore conveniently sterile and pyrogen free. They may also more preferably be incorporated into a form suitable for oral administration either as a liquid or solid.

The peptides may be formulated in a form suitable for oral administration and thus may be incorporated into a liquid, diluent or carrier, although it is more usual to use a solid, for example a conventional solid carrier material such as starch, lactose, dextran or magnesium stearate. Such solid compositions may conveniently be of a formed type, for example as tablets, capsules etc.

The peptide is preferably introduced into the host by a parenteral route, preferably by intraarticular injection. Any of the common liquid or solid vehicles may be employed, which are acceptable to the host and which do not have any adverse side effects on the host or any detrimental effects on the peptide. Phosphate buffered saline (PBS), at a physiological pH, e.g. 6.8 to 7.2, preferably pH 7.0 may be used as a vehicle, alone or with a suitable depot.

The compositions may be formulated in unit dosage form and the amount and frequency of administration being dependent upon the severity of the disease in the individual patient. Constant monitoring of the levels of IgA-$\alpha_1$AT complex for example by the technique described in UKPA 9111215.1 will help to determine the dosage for each particular patient.

The invention will now be illustrated by way of the following Examples. The following registered trade mark are referred to:

Sephadex
Sephacryl
Triton
RPMI 1640

## EXAMPLE 1 - SYNTHESIS OF PEPTIDES

Peptides were synthesised using an LKB Biolynx automated peptide synthesiser using standard Fmoc chemistry. All cysteine residues were protected with trityl groups during synthesis. Cleavage and deprotection of the peptide was performed by using a 95% trifluoroacetic acid (TFA), 5% ethane-dithiol mixture. The peptides were then rotor-evaporated to remove the TFA, followed by an ether extraction to remove the ethane dithiol, using several changes of ether and extracting into 0.05M acetic acid. A final clean up of the peptides was then carried out using a Sephadex Gl0 gel filtration column. Peptides were then freeze dried and stored in a desiccator at 4°C until used. Before use, all peptides were checked for the presence of free SH by a modified Ellman test as follows.

100μl of peptide (0.5mM) was double diluted using 0.1M phosphate buffer (pH 8.0) in a flexible microtitre plate (Falcon). Cysteine (0.2M) was also double diluted to act as a standard. 25μl of 5,5'-dithiobis(2-nitrobenzoic acid), 645μg/ml, was then added to each well. Colour was allowed to develop for 15 minutes, and the plate then read at 410nm in a Multiscan plate reader. A calibration curve was constructed using the cysteine and the free SH for the peptides was calculated.

The following groups of peptides were synthesised, for the purposes of showing the effect of the positioning of the positively charged amino acid residues in relation to the thiol-active cysteine residue on the properties of the peptide, and for comparing peptides having differing sub-generic formulae.

(a) GROUP 1 :

In this group of peptides, there are positively charged amino acid residues on both the N-terminal or C-terminal of the thiol-active cysteine residue. The peptide may be amidated (represented by "nh$_2$") and the positively charged N-terminal residue may be separated from the thiol-active cysteine residue by a spacer residue.

(b) GROUP 2 :

In this group of peptides, the positively charged amino acid residues are located on the C-terminal of the thiol-active cysteine residue.

(c) GROUP 3 :

The peptides in this group all have as a "core" the sequence His Cys Lys Lys. Additionally, the peptide is elongated at the N-terminal by a residue or residues. The effect of the presence of negatively charged residues is investigated.

(d) GROUP 4 :

The peptides of group 4 are as group 3 except the peptide is elongated at the C-terminal by a residue or residues. Again, the effect of negatively charged residues is investigated.

(e) Group 5 :

Various control peptides and peptides which conform to the general formula of the invention (identified by *).

EXAMPLE 2 - PREPARATION OF IgA-$\alpha_1$AT COMPLEX

IgA myeloma (Brierley) plasma was defibrinated by the addition of thrombin (10 IU/100ml plasma). The plasma was then stirred overnight at 4°C and the resulting fibrin clot removed, to give serum. Saturated ammonium sulphate was added dropwise with constant stirring to the serum to a final concentration of 50% at 4°C. The mixture was then centrifuged in an MSE Coolspin 2 at 3000rpm for 15 minutes at 4°C. The resulting precipitate was then redissolved in ultra-pure water and dialysed against several changes of phosphate buffered saline, pH 7.2 (PBS). The sample was then applied, at 4°C, to a Sephacryl S300HR gel filtration column, equilibrated with PBS, and the protein fractions collected, using PBS as the buffer during the separation. Column fractions were assayed for the presence of IgA-$\alpha_1$AT using the ELISA technique with plates precoated with anti IgA-$\alpha_1$AT. Complex-positive fractions were pooled and concentrated using an Amicon Centri-prep concentrator. The complex-rich fractions were then added, at 4°C, to a Sephacryl S200HR gel filtration column and using the ELISA technique, IgA-$\alpha_1$AT positive fractions were collected. Fractions were concentrated as before. Purity was confirmed by HPLC, SDS-PAGE and 2-dimensional electrophoresis.

EXAMPLE 3 - IN VIVO STUDIES

(a) Methods of inducing rheumatoid arthritis in rabbits

(i) Active Model

Active models of RA may be set up in rabbits by the classical Dumonde D.C. - Glynn L.G. procedure (British Journal of Experimental Medicine, 1962, 43 p373) or by a variation of this method which involves the use of altered self-IgG as the arthritogen to initiate the formation of rheumatoid factor not seen in the classical model, (Galloway et al., Immunology, 1983, 49, 511). An alternative method described below was carried out.

Rabbits were injected subcutaneously (sc) with 5mg ovalbumin (OA) in 0.5ml sterile saline emulsified in 0.5ml complete Freunds adjuvant (CFA) in 3 separate sites in the scruff of the neck. 14 days later a dose of 5mg OA in 0.5ml sterile saline emulsified in incomplete Freunds adjuvant (IFA) was injected subcutaneously into 3 sites in the scruff of the neck. After a further 10 days the animals were tested for a delayed hypersensitivity response by the intradermal injection of OA (100μl) at 500, 200, 100 mg/ml in sterile saline, with sterile saline used as a negative control. After 24 hrs. the sites of injection were examined and any swelling and redness assessed. All animals that had a negative response (no visible swelling and little or no redness) were given another injection (subcutaneously) of 5mg OA in sterile saline emulsified in 0.5ml IFA. 7 days later, arthritis was induced by the intra-articular injection of 10mg OA In 0.5ml sterile saline into the right knee joint, 0.5ml of sterile saline being injected into the left knee joint as a negative control. The degree of swelling was monitored by the measurement of knee width, using calipers.

(ii) Passive Model

Arthritis was induced in groups of rabbits by the injection of isolated IgA-$\alpha_1$AT complex into their knee joints. IgA-$\alpha_1$AT complex was purified by the method described in Example 2. This results in the rapid development of chronic arthritis in the rabbits. This system has two advantages over the active model, firstly, the speed of RA development and secondly the knowledge that the RA has been induced by the IgA-$\alpha_1$AT complex itself. This is demonstrated as follows:

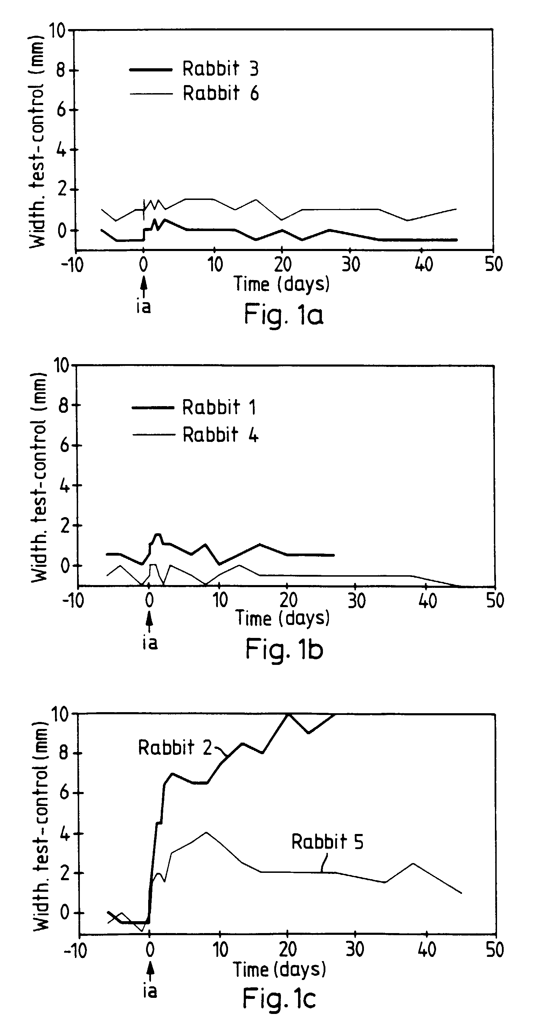

Pairs of normal rabbits were injected intra-articularly (ia) with 0.5ml saline (negative control) in one joint and 0.5ml test substance (IgA, IgA-$\alpha_1$AT complex or synovial fluid (SF) which was negative for IgA-$\alpha_1$AT complex but high in $\alpha_1$AT) in the other. The progression of RA was monitored by measuring the joint width using callipers, comparing the control and test substance injected joints, (Fig. 1a, b and c).

At the end of the experimental period the joints were examined macroscopically and histopathalogically. Macroscopically the IgA and SF injected joints had a normal appearance, no swelling visible, the supra and infra patella fat pads were white with no overgrowth of patella by fat pads, whereas the IgA-$\alpha_1$AT complex injected animals were visibly swollen and the fat pads were brown (indicating cellular infiltration) enlarged and overgrowing the patella and crusiate ligaments. The histopathology showed cellular infiltration into the fat pads, increased number of cells in the synovium lining layer, villus formation, overgrowth of the patella and destruction of the proteoglycan in the patella. All changes seen in the IgA-$\alpha_1$AT complex-injected animals were consistent with a rheumatoid arthritis-type of disease.

(b) Measuring the anti-rheumatic potential of the peptides in passive model of RA

Normal rabbits were injected intra-articularly (ia) with either 0.5ml saline in the left knee as negative control, or 0.5ml purified IgA-$\alpha_1$AT complex in the right knee to induce the arthritis. This induced an immediate arthritis which was monitored by measuring the joint width with callipers. 48 hours after the induction of the arthritis, 0.1ml of a 10mg/ml solution of the peptide His-Cys-Lys-Lys, representing amino acids 231-234 of $\alpha_1$AT were injected ia. As a negative control other animals were treated with oral lactose. This was repeated at 2-3 day intervals over the test period. After 14 days the animals were sacrificed and joint sections taken for later histological examination.

| Day 0 | saline (left knee), IgA-$\alpha_1$AT (right knee) |
|---|---|
| Day 2 | peptide (right knee) |
| Day 4 | "    "    " |
| Day 7 | "    "    " |
| Day 9 | "    "    " |
| Day 11 | "    "    " |
| Day 14 | animals sacrificed |

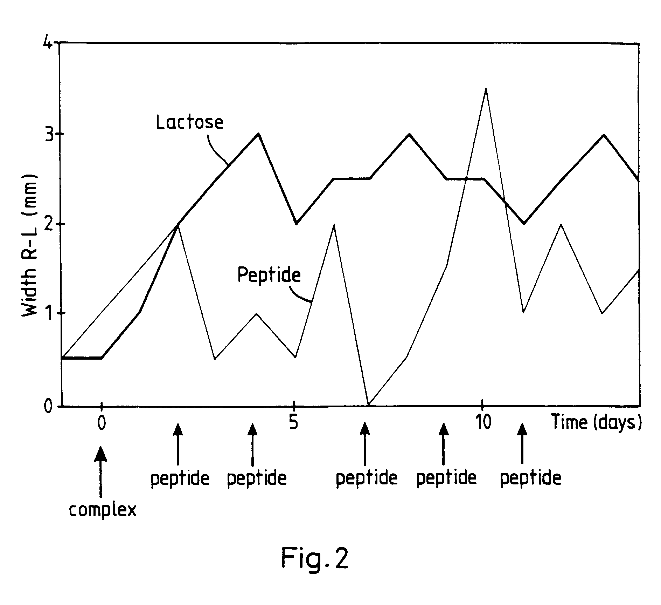

The results are shown in Figure 2.

Statistical (t-test) analysis of the data revealed a significant decrease of swelling of the joints of the peptide injected group on two different occasions throughout the treatment period (i.e. days 1 and 11 after start of treatment); by comparison with the size of joints of animals in the negative control group (treated with placebo).

EXAMPLE 4 - IN VITRO STUDIES OF ANTI-RHEUMATIC ACTIVITY OF SYNTHETIC PEPTIDES

The ability of the peptides of the invention to dissociate purified IgA-$\alpha_1$AT complex in vitro was tested as described below. The anti-rheumatic drug D-penicillamine and other synthetic peptides were used as controls.

<u>(a) HPLC ANALYSIS</u>

50μl of a 57 μg/ml solution of purified IgA-$\alpha_1$AT complex was incubated with an equal volume of peptide or drug (500μM) for 90 minutes at 37°C. 25μl of the incubated mixture was then applied to a 30cm long TSK G3000 SWXL HPLC column. The sample was monitored at wavelength 206nm at a flow rate of 1ml/minute with phosphate buffer (0.1M) pH 7.1.

The results are shown in the column headed "HPLC minimum" in Tables 1-5 below. They represent the ability of the peptides to dissociate the IgA-$\alpha_1$AT complex <u>in</u> <u>vitro.</u>

In these experiments, the base-lines level comprises a complex : free IgA ratio of 14% . The minimum concentration of peptide taken to produce a positive dissociation effect is that which results in a complex: free IgA ratio at 20%. In the results tables, all the peptide values are expressed in μMoles. Peptide values at >500μM are taken to indicate that no complex dissociation occurred at the highest peptide concentration tested.

<u>(b) INHIBITION OF C3-ACTIVATION</u>

Various peptides were tested for their ability to prevent the IgA-$\alpha_1$AT complex from activating the alternative complement (C3) pathway.

The method of measuring the capacity to activate the alternative complement pathway by the non-immune IgA-$\alpha_1$AT complex and the subsequent inhibition of this activation by anti-rheumatic peptides is described below.

This method was based on the method described by Riches and Stanworth, Immunology Letters, 1980, 1, 363,366, with modification to miniaturise it thus allowing the assay to be carried out on microtitre plates.

The initial observation by Platts-Mills and Ishizaka (J Immunol. (1974) <u>113</u>, 348,358) that low dilutions of fresh normal human serum, from which calcium had been removed by chelation with 10mM EGTA to block the classical complement pathway, caused greater than 90% haemolysis of unsensitised rabbit erythrocytes when incubated at 37°C was employed in this assay.

The non-immune complex IgA-$\alpha_1$AT had been shown in the past to be the only known soluble complex to activate the alternative pathway complement and inhibit the lysis of the rabbit erythrocytes. This inhibition required biologically active complex. Complex isolated from 10 year old IgA myeloma plasma was found to be unable to activate alternative complement and hence was not biologically active.

<u>Buffers Required</u>

The buffers solutions required in this assay were (a) Total lysis buffer (1% Triton100 in ultra pure water), (b) Alternative pathway complement fixation diluent (APCFD) which was:-

83 volumes CFD, DH 7.2 (1 oxoid tablet: 100ml UPW)
10 volumes 0.1M EGTA pH 7.2
7 volumes MgCl$_2$ pH 7.2

This buffer was also prepared in a double concentrated form, and (c) cell storage buffer (0.1M citrate buffer pH 6.2).

<u>Serum</u>

Blood was obtained by venipuncture from a healthy human individual. The blood was allowed to clot for 1 hour at room temperature and then stored overnight at 4°C. The serum was removed and stored at -70°C in lml aliquots. For use, the serum was defrosted and 100μl of 0.1m EGTA pH 7.2 and 70μl O.lm MgCl$_2$ pH 7.2 was added.

<u>Rabbit Red Blood Cells</u>

Rabbit red blood cells (RBC) were obtained by venipuncture of a healthy rabbit and collected into 0.1M citrate buffer pH 6.2. The RBC were washed 3 times in citrate buffer and the cells were stored in this buffer which was changed every week. It was possible to store cells in this way for 3 weeks. <u>Peptides</u>

The peptides were synthesised as described in Example 1 and made up in UPW to lmM. They were kept frozen at -70°C prior to use.

IgA-$\alpha_1$AT Complex

This was prepared as previously described in Example 2. It was kept frozen in small aliquots prior to use.

METHOD OF PERFORMING THE ASSAY

(i) Estimation of dilution of serum needed to just lyse 100% of Rb RBC:

Using a round bottomed welled flexible microtitre plate 100µl neat serum (with added EGTA and MgCl$_2$) was added to the first wells in duplicate. This was double diluted out, with APCFD to give a volume of 50µl well. 8 test wells (+ duplicates) were sufficient.
8 wells were prepared as a positive control - (100µl 1% Triton/UPW).
8 wells were prepared as a negative control - (100µl APCFD (Blank)).
50µl of APCFD was added to the test wells and 50µl of 1% rabbit RBC was added to all wells. The plate was then incubated at 37°C for 30 minutes and then centrifuged for 10 minutes at 2000 rpm.
75µl of the supernatant was carefully removed and plated onto a flat bottom welled microtitre plate. The plates were then read at 410nm interference, 690nm reference. The zero or blank value was calculated as the mean value of negative control wells and using the mean of the positive control wells, the percentage lysis for serum dilutions was calculated. In future assays the serum dilution in which lysis was approx. 80% of Rb RBC was used. This was usually in the range 1:4 - 1:6 dilution (ie. lml serum : 4ml APCFD - 6ml APCFD).

(ii) Using IgA-$\alpha_1$AT complex as an alternative comDlement pathway inhibitor

A 1:1 dilution of IgA-$\alpha_1$AT complex was made with twice concentrated APCFD. 100µl of this 1:1 dilution of complex was added to the first wells in duplicate. This was double diluted out with APCFD to give 50 µl per well. 50µl of the predetermined dilution of serum (see (i) above) was added to each well. Positive and negative control wells as described in (a) above and wells containing serum alone were used as controls. The plates were incubated at 37°C for 30 minutes and then centrifuged at 2000 rpm for 1 minute. 50µl of 1% rabbit red blood cells were added to each well and the plates were incubated again at 37°C for 30 minutes. The plate was then re-centrifuged at 2000 rpm for 10 minutes. 75µl of the supernatant was removed from each well and plated on to a flat bottomed welled microtitre plate which was read at 410nm interference 690nm reference.
The control wells were checked to ensure that the dilution of serum used had lysed the rabbit RBC to a significant amount. Then, the % inhibition of the mean values for the complex dilutions were calculated with respect to the serum control wells. This enabled a suitable concentration of IgA-$\alpha_1$AT complex to be determined for the next stage of the assay. A suitable concentration was in the range 1:1 - 1:4 complex : APCFD.

(iii) Use of putative anti-rheumatic peptides as inhibitors of complex inhibition of alternative pathway complement lysis of Rabbit RBC.

The peptides (500µH concentration) were diluted 1:1 with the double concentrated APCFD. These solutions of peptides were then double diluted down a round bottomed welled microtitre plate, such that there was 25µl of peptide solution per well. Positive and negative controls, serum only controls and serum plus IgA-$\alpha_1$AT complex controls were included in the assay. 25µl of a suitable dilution of IgA-$\alpha_1$AT complex (see (iii) above) was added to the test wells and the plates were centrifuged at 2000rpm for 1 minute. The plates were then incubated at 37°C for 1½ hours. 50µl of a suitable serum dilution (see (a) above) was added and the plates centrifuged at 2000 rpm for 1 minute, then incubated at 37°C for 30 minutes. 50µl of 1% rabbit RBC was added and after a further 30 minutes of 37°C incubation, the plates were centrifuged at 2000 rpm for 10 minutes. Supernatants were taken as previously.

Interpretation of Results

The mean values of each of the serum(s), serum and complex (c + s) and total haemolysis wells were calculated. The % haemolysis with respect to lysis by serum wells was calculated. The peptide was considered to have titred out when % lysis value was equal to the calculated % lysis value given by the c + s mean. The titre point for the peptide was calculated as the concentration in µM of the peptide that just gave greater lysis than the c + s mean value.
The results are shown in Tables 1-5 below. The results are expressed as the minimum concentration of peptide needed to prevent C3 activation, and are found in the column headed "C'".

## (c) MACROPHAGE INHIBITION ASSAY

A mouse macrophage cell line (PU 518) was stimulated to release lysosomal enzymes by the addition of purified human IgA-α1AT complex. The degree to which this release was then inhibited by the peptides or other drugs was investigated, with the degree of inhibition being indicative of activity; the concentration of peptide or other test drugs giving 50% inhibition being recorded. Briefly the method was as follows:

25μl of peptide or drug was double diluted in RPMI 1540 + 10% foetal calf serum (FCS) in a sterile tissue culture 96 well plate. 25μl of IgA-$\alpha_1$AT complex in RPMI 1640 +10% FCS at about 0.2mg/ml was then added to all wells. 50μl of cells at a concentration of $2 \times 10^6$ cells/ml in RPMI 1640 + 10% FCS was then added to each well.

Control wells of cells alone and cells + IgA-$\alpha_1$AT complex were also prepared. The plate was then gently mixed and incubated overnight (16hr) at 37°C in a 4% $CO_2$ atmosphere. Plates were then centrifuged at 1200 rpm for 10 minutes in a bench top centrifuge.

40μl of supernatant was then taken from each well and added to a 96 well polystyrene assay plate, to which was added 40μl of 160mM acetate buffer (pH 4.3) containing 2mM phenolphthalein-β-D-glucuronic acid. To obtain a standard curve, dilutions of phenolphthalein in acetate buffer from 100-5μg/ml were also added to separate wells in the assay plate.

The plates were then incubated at 37°C for 4 hours before 150μl of 0.2M glycine/NaOH buffer (pH 10.6) containing 0.2M NaCl was added to stop the reaction.

Plates were then shaken for 3 minutes before the optical density at 570nm was read on a plate reader, and the amount of β-D-glucuronic acid released from the cells calculated. The concentration of peptide or drug giving 50% inhibition of release was then noted.

The results for the macrophage inhibition assays are expressed as the concentration needed to prevent 50% release of β-glucoromidase.

The results of these experiments are tabulated in Tables 1 - 5 below.

TABLE 1 :

| GROUP 1 PEPTIDES | | | | |
|---|---|---|---|---|
| Sequence | Peptide number (for Ref) | HPLC % (μM) | C' (μM) | Macrophage 50% Inhibition (μM) |
| HCKKnh$_2$ | 132 | 10 | 16 | 60 |
| HDCKKnh$_2$ | 198 | 10 | 3.9 | |
| HYCKKnh$_2$ | 199 | 10 | 3.9 | |
| HPCKKnh$_2$ | 201 | 10 | 3.9 | |
| HECKKnh$_2$ | 204 | 10 | <1.95 | |
| HFCKKnh$_2$ | 208 | 10 | 3.9 | |
| HFCKKnh$_2$ | 209 | 10 | 3.9 | |

TABLE 2 :

| GROUP 2 PEPTIDES | | | | |
|---|---|---|---|---|
| | Peptide number(s) | HPLC% minimum (μM) | C' minimum (μM) | Macrophage 50% Inhibition |
| Sequence | | | | |
| CKKnh$_2$ | 114 | 125 | >1250 | |

TABLE 3 :

| GROUP 3 PEPTIDES | | | | |
|---|---|---|---|---|
| Sequence | Peptide number(s) | HPLC (μM) | C' (μM) | macrophage 50% inhibition (μM) |
| DHCKKnh$_2$ | 113 | 10 | 78 | 70 |
| EHCKKnh$_2$ | 154 | 5 | <2 | |
| DGHCKKnh$_2$ | 119 | 10 | | |
| EGHCKKnh$_2$ | 155 | 5 | <2 | 60 |

TABLE 3 : (continued)

| GROUP 3 PEPTIDES | | | | |
|---|---|---|---|---|
| Sequence | Peptide number(s) | HPLC (μM) | C' (μM) | macrophage 50% inhibition (μM) |
| DGGHCKKnh$_2$ | 118 | 50 | | |
| dAHCKKnh$_2$ | 205 | 10 | 1.95 | |
| QHCKKnh$_2$ | 133 | 10 | 31 | |
| IQHCKKnh$_2$ | 134 | 10 | 62 | |
| NIQHCKKnh$_2$ | 135 | 10 | 31 | |
| (d denotes D-residues of amino acids) | | | | |

The mixed charge constructs were active.

TABLE 4 :

| GROUP 4 PEPTIDES | | | | |
|---|---|---|---|---|
| Sequence | Peptide number(s) | HPLC (μM) | C' (μM) | macrophage 50% inhibition (μM) |
| HCKKDnh$_2$ | 120 | 50 | 62 | 25 |
| HCKKEnh$_2$ | 150 | 50 | 8 | |
| HCKKGDnh$_2$ | 121 | 125 | 62 | |
| HCKKGEnh$_2$ | 151 | 50 | 16 | |
| HCKKGGDnh$_2$ | 122 | 50 | 31 | |
| HCKKGGEnh$_2$ | 152 | 10 | 125 | |
| HCKKdAnh$_2$ | 206 | 50 | 31.3 | |
| (d denotes D-residues of amino acids) | | | | |

The mixed charge constructs were active.

TABLE 5 :

| GROUP 5 PEPTIDES | | | | |
|---|---|---|---|---|
| Sequence | Peptide Number | HPLC minimum (μM) | C' minimum (μM) | Macrophage 50% inhibition |
| D-Penicillamine | - | 250 | 31 | 500 |
| Cysteine | - | 250 | 16 | 500 |
| EVDGTCY | 124 | 250 | 16 | |
| VDGTCY | 125 | 250 | 31 | |
| DGTCY | 126 | >500 | | |
| GTCY | 127 | 50 | | |
| TCY | 128 | 125 | | |
| Substance P | 36 | >500 | >500 | >500 |
| HCKK* | 107 | 250 | | |
| HCK | 110 | 500 | | |
| HC | 112 | 250 | | |
| CK | 109 | 500 | | |
| CKK * | 106 | 500 | | |
| CKnh$_2$ | 115 | 500 | | |
| Glutathione | - | 500 | 31 | |
| N-acetyl Cysteine | - | 500 | 4 | |

(d denotes D-residues af amino acids)
   (* denotes a peptide of the invention)

TABLE 5 :　(continued)

| GROUP 5 PEPTIDES | | | | |
|---|---|---|---|---|
| Sequence | Peptide Number | HPLC minimum (µM) | C' minimum (µM) | Macrophage 50% inhibition |
| KTKGSGFFVF | 30 | >500 | 31 | 250 |
| VSVVMAEVDGTCY | 80 | 10 | 16 | |
| IVLVDNKCKCAR | 89 | 50 | 125 | |
| GMFNIQHCKKLSS | 90 | 50 | 31 | |
| HCCGKnh$_2$ | 147 | 10 | 32 | 250 |
| KAAGS | 96 | >500 | | |
| DHCKK * | 108 | 10 | | |
| dAHCKKdAnh$_2$ | 210 | 10 | 3.9 | |

(d denotes D-residues af amino acids)
(* denotes a peptide of the invention)

Extension of the core tetrapeptide (His Cys Lys Lys) sequence as in peptides 133-135 had no effect on activity. Smaller versions of the core tetrapeptide His Cys Lys Lys (106, 109, 110, 112, 115) were substantially less active; as was a non-amidated form (107) of the core tetrapeptide. Amongst the control reducing substances tested, glutathione and N-acetyl cysteine were relatively inactive.

## Claims

1.　A pharmaceutical composition comprising a prophylactically or therapeutically effective amount of a synthetic peptide consisting of from 3 to 7 amino acid residues or analogue thereof wherein the analogue is at least partly non-peptide in nature comprising a single thiol-active cysteine residue and having the following general formula:

$$Z_a^1\text{-}B_c^1\text{-}Cys\text{-}B_d^2\text{-}Lys\text{-}Lys\text{-}Z_b^2$$

Wherein a positively charged amino acid residue is located on the peptide's N-terminal or C-terminal; and wherein $Z^1$, $Z^2$, $B^1$ and $B^2$ represent sequences of positively charged, negatively charged or neutral amino acid residues or sequences of any mixture of positively charged, negatively charged or neutral amino acid residues; or $B^1$ and $B^2$ represent non-peptide spacer arms of a length equivalent to that determined by the length of d and c residues of amino acids
c = 0 to 4, d = 0 to 4; and
a = 0 to 4 and b = 0 to 4 and a + b + c + d ≤ 4;
the peptide or analogue thereof being capable of preventing the formation of or dissociating already formed IgA-$\alpha_1$AT complex, with the proviso that the peptide does not include the sequence Cys-Ala-Lys-Lys-Ile or Cys-Lys-Lys-Thr-Glu.

2.　A composition according to claim 1 wherein d=0 so that the positively charged Lys-Lys residue is directly adjacent to the thiol-active cysteine residue.

3.　A composition according to claim 1 wherein d≠0 so that the positively charged Lys-Lys residue is separated from the thiol-active cysteine residue by a spacer arm.

4.　A composition according to claim 3 wherein the spacer arm comprises 1-4 amino acid residues.

5.　A composition according to claim 4 wherein the amino acid residues are neutral amino acid residues.

6.　A composition according to claim 5 wherein the neutral amino acid residues are glycine.

7.　A composition according to claim 1 consisting of the residues Cys-Lys-Lys.

8.　A composition according to claim 1 consisting of the residues His-Cys-Lys-Lys.

EP 0 614 464 B1

**9.** A composition according to any preceding claim which is amidated at the C-terminal.

**10.** A pharmaceutical composition according to claim 1 which is suitable for injection.

**11.** A pharmaceutical composition according to claim 1 which is suitable for oral administration.

**Patentansprüche**

**1.** Eine pharmazeutische Verbindung enthaltend eine prophylaktisch oder therapeutisch wirksame Menge eines synthetischen Peptids, bestehend aus 3 bis 7 Aminosäureresten oder einem Analogon davon, worin das Analogon zumindest teilweise nicht-peptidischer Art ist, einen einzigen Thiol-aktiven Cysteinrest enthält und die folgende allgemeine Formel besitzt:

$$Z^1_a\text{-}B^1_c\text{-}Cys\text{-}B^2_d\text{-}Lys\text{-}Lys\text{-}Z^2_b$$

worin ein positiv geladener Aminosäurerest an dem End-N oder End-C des Peptids liegt; und worin $Z^1$, $B^1$ und $B^2$ Sequenzen von positiv geladenen, negativ geladenen oder neutralen Aminosäureresten oder Sequenzen von irgendeiner Mischung von positiv geladenen, negativ geladenen oder neutralen Aminosäureresten darstellen oder worin $B^1$ und $B^2$ nicht-peptidische Seitenkettenarme mit einer Länge darstellen, die äquivalent zu der Länge ist, die von Resten d und c von Aminosäuren bestimmt wird,
c=0 bis 4, d=0 bis 4; und
a=0 bis 4 und b=0 bis 4 und $a + b + c + d \leq 4$;
wobei das Peptid oder Analogen davon zur Verhinderung der Bildung oder zur Dissozilerung eines bereits gebildeten IgA-$\alpha_1$AT-Komplexes fähig ist, mit dem Vorbehalt, daß das Peptid nicht die Sequenz Cys-Ala-Lys-Lys-Ile oder Cys-Lys-Lys-Thr-Glu einschließt.

**2.** Eine Verbindung gemäß Anspruch 1, worin d ≠ 0, so daß der positiv geladene Lys-Lys-Rest direkt an den Thiol-aktiven Cysteinrest angrenzt.

**3.** Eine Verbindung gemäß Anspruch 1, worin d ≠ 0, so daß der positiv geladene Lys-Lys-Rest von dem Thiol-aktiven Cysteinrest durch einen Seitenkettenarm getrennt ist.

**4.** Eine Verbindung gemäß Anspruch 3, worin der Seitenkettenarm 1 - 4 Aminosäurereste enthält.

**5.** Eine Verbindung gemäß Anspruch 4, worin die Aminosäurereste neutrale Aminosäurereste sind.

**6.** Eine Verbindung gemäß Anspruch 5, worin die neutralen Aminosäurereste Glycin sind.

**7.** Eine Verbindung gemäß Anspruch 1, bestehend aus den Resten Cys-Lys-Lys.

**8.** Eine Verbindung gemäß Anspruch 1, bestehend aus den Resten His-Cys-Lys-Lys.

**9.** Eine Verbindung gemäß irgendeinem vorhergehenden Anspruch, welche an dem End-C amidiert ist.

**10.** Eine pharmazeutische Verbindung gemäß Anspruch 1, welche für eine Injektion geeignet ist.

**11.** Eine pharmazeutische Verbindung gemäß Anspruch 1, welche für eine orale Verabreichung geeignet ist.

**Revendications**

**1.** Composition pharmaceutique comprenant une quantité prophylactiquement ou thérapeutiquement efficace d'un peptide synthétique constitué de 3 à 7 résidus acides aminés ou d'un de ses analogues, dans laquelle l'analogue est de nature au moins partiellement non peptidique, comprenant un seul résidu cystéine thiol-actif et ayant la formule générale ci-dessous:

$$Z^1{}_a - B^1{}_c - Cys - B^2{}_d - Lys - Lys - Z^2{}_b$$

dans laquelle un résidu acide aminé positivement chargé est situé sur l'extrémité N-terminale ou C-terminale du peptide; et dans laquelle $Z^1$, $Z^2$, $B^1$ et $B^2$ représentent des séquences de résidus acides aminés positivement chargés, négativement chargés ou neutres ou des séquences de n'importe quel mélange de résidus positivement chargés, négativement chargés ou neutres; ou $B^1$ et $B^2$ représentent des bras espaceurs non peptidiques d'une longueur équivalente à celle qui est déterminée par la longueur des résidus c et d des acides aminés
c = 0 à 4, d = 0 à 4; et
a = 0 à 4 et b = 0 à 4 et a + b + c + d ≤ 4;
le peptide ou son analogue étant capable d'empêcher la formation de ou de dissoudre un complexe IgA-$\alpha_1$AT , sous réserve que le peptide n'inclut pas la séquence Cys-Ala-Lys-Lys-Ile ou Cys-Lys-Lys-Thr-Glu.

**2.** Composition selon la revendication 1 dans laquelle d est différent de zéro si bien que le résidu Lys-Lys positivement chargé est directement adjacent au résidu cystéine thiol-actif.

**3.** Composition selon la revendication 1 dans laquelle d est différent de zéro si bien que le résidu Lys-Lys positivement chargé est séparé du résidu cystéine thiol-actif par un bras espaceur.

**4.** Composition selon la revendication 3 dans laquelle le bras espaceur comprend de 1 à 4 résidus acides aminés.

**5.** Composition selon la revendication 4 dans laquelle les résidus acides aminés sont des résidus acides aminés neutres.

**6.** Composition selon la revendication 5 dans laquelle les résidus acides aminés neutres sont de la glycine.

**7.** Composition selon la revendication 1 constituée des résidus Cys-Lys-Lys.

**8.** Composition selon la revendication 1 constituée des résidus His-Cys-Lys-Lys.

**9.** Composition selon l'une quelconque des revendications précédentes qui est amidée à l'extrémité C-terminale.

**10.** Composition pharmaceutique selon la revendication 1 qui convient pour l'injection.

**11.** Composition pharmaceutique selon la revendication 1 qui convient pour l'administration orale.

Fig. 1a

Fig. 1b

Fig. 1c

Fig.2